# EUROPEAN PATENT APPLICATION

(11) **EP 4 837 511 A1**
(43) Date of publication of application: **11.11.2026**
(21) Application number: 26175843.7
(22) Date of filing: 30.04.2026
(51) Int. Cl.: A61B 6/50

(54) **CT IMAGING SYSTEM FOR LARGE ANIMALS**

(30) Priority: 05.05.2025 BE 202505291
(71) Applicant: Medical Imagery SA, 4950 Waimes (BE)
(72) Inventor: BULTOT, Dominique, 4950 Waimes (BE)
(74) Representative: V.O.

(57) **Abstract**

The present disclosure relates to a computed tomography imaging system for imaging a body part of a large animal, such as a horse. The CT imaging system comprises a CT gantry and a mobile positioning device for positioning the large animal. The CT imaging system comprises a first elevator mechanism configured for moving the CT gantry in a vertical direction, between a lower gantry position and an upper gantry position, such that a height of the isocenter of the CT gantry relative to a floor surface is variable. The mobile positioning comprising at least a platform configured for supporting the large animal in a standing position and in a recumbent position, a second elevator mechanism configured for moving the platform in the vertical direction between a lower platform position and an upper platform position, such that a height of the platform with respect to the floor surface is variable, and a longitudinal movement mechanism configured for moving the platform in the longitudinal direction.

## Description

### FIELD OF THE INVENTION

The invention relates to a computed tomography (CT) imaging system for imaging a body part of a large animal, such as for example a horse.

### BACKGROUND OF THE INVENTION

Computed tomography (CT) imaging has become an essential diagnostic tool in veterinary medicine, particularly for large animals such as horses. However, adapting conventional CT scanners for equine imaging presents significant challenges due to the size, weight, and anatomical structure of these animals.

A first type of CT scanner, as used in human medicine, may consists of a fixed CT gantry with an annular opening through which a motorized patient table moves longitudinally during image acquisition. For taking a CT scan, the patient is lying on the table in a horizontal position. For image acquisition, the CT gantry generally houses at least an X-ray source for emitting an X-ray fan beam towards the part of the body to be imaged and a detector for detecting the X-rays having crossed the body part. By rotating the X-ray source and detector around the body part a plurality of cross-sectional images are taken from different directions, and which may be reconstructed into a 3D image. By moving the patient table through the gantry opening during image acquisition, a full-body image may be taken. Hence, with this type of CT scanner, during imaging, the CT gantry remains stationary.

A second type of CT scanner for human medicine which comprises a so-called sliding CT gantry moveable on rails, is disclosed in for example patent document US2025/0057489. With this type of CT scanner, for taking a CT scan of the patient in supine position, the patient is resting on a patient table in a horizontal position and, while the table remains stationary, the CT gantry is moved along the rails and the gantry ring is sliding over the patient.

However, standard commercial CT scanner designs are not well suited for large animals for several reasons. First, size constraints prevent an entire horse from fitting within the scanner's bore, requiring adaptations for partial imaging. Second, horses and other large animals cannot lie still for extended periods without general anesthesia, which is both risky and logistically complex. Third, weight limitations on standard patient tables make it impractical to support large animals. As a result, traditional CT scanners require significant modifications to be used effectively in equine and large-animal imaging.

To address these challenges, specialized CT scanner systems have been developed that modify the conventional design to accommodate for large animals.

An example of CT imaging system developed for large animals is described in patent document EP3668405B1, which presents a solution for scanning specific body parts of a standing horse, such as the head or feet. The system uses a commercially available CT gantry and a classical CT patient table. The system comprise two platforms: a first platform moving the entire assembly of table and gantry, up and down, and a second platform on which the gantry slides. In other words, this type of CT scanner is not configured as a fixed CT scanner but as the so-called sliding gantry wherein during image acquisition the patient remains stationary while the gantry makes a longitudinal motion. In this system, during image acquisition, the horse remains standing on a stationary floor in front of a first side of the gantry and only the body part requiring imaging, for example the head of the limb of the horse, is resting on the classical CT table which is located on a second side of the gantry, opposite the first side. Hence, with this system, a classical patient table for humans is used for supporting only a portion of the animal, i.e. the body part to be imaged, and the classical patient table remains stationary during performance of a CT scan.

A disadvantage of this sliding gantry system is the elevated price and that complex controls are required for longitudinally moving both the platform supporting the gantry and the classical CT table during imaging. Indeed, to ensure that the scanned body part resting on the classical CT table remains stationary during imaging, the classical CT table has to move synchronously in the opposite direction of the moving gantry. Hence, a sliding gantry-platform system introduces mechanical complexity and necessitates precise synchronization between the gantry's longitudinal motion and the opposite movement of the CT table. Any misalignment in this system can lead to image artifacts, reducing diagnostic accuracy.

A second disadvantage of the system described in EP3668405B1 is that horses under anesthesia cannot be treated, as with this CT scanner system, during imaging, the horse always has to stand or at least partly stand on his feet. Unless if the horse is laying on the ground, which has major drawbacks. Furthermore, the assembly is located in a pit, requiring the user to climb down into it and which adds risks of falling.

Hence, there is room for improving CT imaging systems for large animals.

### SUMMARY OF THE INVENTION

It is an object of the present disclosure to provide a practical, compact and cost-effective solution for CT imaging of horses, allowing imaging of both standing and anesthetized horses, or large animals in general.

The present disclosure is defined in the appended independent claims. The dependent claims define advantageous embodiments.

According to a first aspect of the invention, a computed tomography (CT) imaging system for imaging a body part of a horse, is provided.

The wording "CT imaging system", "CT scanner" and "CT scanner system" are synonymous and have been used interchangeably throughout the present disclosure.

The CT imaging system comprises a CT gantry having an annular opening for receiving the body part to be imaged and/or examined. A central axis of the CT gantry extending through the annular opening is crossing an isocenter of the CT gantry, and the central axis is parallel with a longitudinal direction. The CT gantry comprises at least an X-ray source and a detector for detecting X-rays.

The CT imaging system further comprises a first elevator mechanism configured for moving the CT gantry in a vertical direction, transverse to the longitudinal direction, between a lower CT gantry position and an upper CT gantry position, such that a height of the isocenter relative to a floor surface is variable.

The CT imaging system further comprises a mobile positioning device for positioning the horse. The mobile positioning device comprises at least: a) a platform configured for supporting the large animal in a standing position and in a recumbent position, b) a second elevator mechanism configured for moving the platform in the vertical direction between a lower platform position and an upper platform position, such that a height of the platform with respect to the floor surface is variable, and c) a longitudinal movement mechanism configured for moving the platform in the longitudinal direction. The platform has a platform length measured in the longitudinal direction that is equal or larger than 1.8 m, preferably equal or larger than 2 m, and wherein the platform has a platform width measured in a lateral direction perpendicular to the longitudinal direction that is equal or larger than 1.2 m, preferably equal or larger than 1.5 m.

The CT imaging system further comprises a control system configured for taking a CT scan by synchronizing a movement of the platform along the longitudinal direction with a rotational movement of the X-ray source and/or the detector for taking X-rays, preferably wherein the CT scan is a helical CT scan.

Advantageously, by providing a first elevator mechanism for adjusting the gantry isocenter with respect to a floor level and a second elevator mechanism for adjusting a height of the platform supporting the animal, a large number of body parts of for instance a standing horse may be located at the isocenter of the CT gantry and hence be imaged. For example for imaging a head of a standing horse, the platform may be lowered to a low position and the CT scanner may be raised to a high position. On the other hand, for imaging a limb part, such as a foot of a horse, while the horse is in a standing position, the height of the platform may be somewhat raised to a higher position and the CT scanner may be lowered to a lowest position.

Advantageously, the platform is also configured for supporting the large animal in a recumbent position. In this way, by raising the platform to a certain height above the floor level, e.g. 90 cm, the platform is forming a supporting table and body parts of the animal may be imaged while the animal is under anesthesia. In addition, when needed, surgical operations may be handled while the animal is in the recumbent position under anesthesia in front of the gantry.

Advantageously, a commercial CT scanner with a fixed CT gantry may be used, i.e. a CT gantry that does not need to be moved in the longitudinal direction during imaging, as is the case with a sliding CT gantry. Hence with such a CT scanner, the CT gantry remains stationary during imaging, while only the platform is to be moved in the longitudinal direction for taking a CT scan, e.g. a helical CT scan.

Optionally and/or additionally, the first elevator mechanism may have a first travel stroke ΔS1 in the vertical direction for moving the CT gantry between the lower and upper gantry position, and wherein the first travel stroke ΔS1 is equal or larger than 30 cm, preferably equal or larger than 50 cm, more preferably equal or larger than 80 cm.

Optionally and/or additionally, the first elevator mechanism comprises a plurality of screw jacks, for example motorized screw jacks.

Optionally and/or additionally, the second elevator mechanism may have a second travel stroke ΔS2 in the vertical direction for moving the platform between the lower and upper platform position, and wherein the second travel stroke ΔS2 is equal or larger than 70 cm, preferably equal or larger than 80 cm, more preferably equal or larger than 90 cm.

Optionally and/or additionally, the second elevator mechanism comprises a hydraulic lifting device.

Optionally and/or additionally, the longitudinal movement mechanism may have a third travel stroke ΔS3 for moving the platform in the longitudinal direction between a first and a second platform longitudinal position, and wherein said third travel stroke ΔS3 is equal or larger than 50 cm, preferably equal or larger than 70 cm, more preferably equal or larger than 130 cm.

In embodiments, the lower platform position may correspond to a position wherein a top surface of the platform is leveled with the floor surface. In other embodiments, the lower platform position may correspond to a position wherein a top surface of the platform is below the floor surface.

Optionally and/or additionally, the upper platform position may correspond to a position wherein the top surface of the platform is raised by at least 70 cm, preferably by at least 80 cm, more preferably by at least 90 cm above the floor surface.

Optionally and/or additionally, the floor surface may be a surface associated to a floor of a room wherein the computed tomography imaging system is located.

Optionally and/or additionally, the first and second elevator mechanism are located in a pit in the floor of the room.

Optionally and/or additionally the mobile positioning device may be located in front of the CT gantry.

Optionally and/or additionally, the height of the platform with respect to the floor surface may be measured with respect to the top surface of the platform.

Advantageously, the first and second elevator mechanism may be configured for operating independently from each other such that the height of the isocenter of the CT gantry and the height of the platform are independently adjustable.

In other words, by utilizing independent elevators for adjusting both the gantry height and the table height, the system enhances positional flexibility, reduces mechanical complexity, and minimizes space requirements, offering a practical and cost-effective solution for equine CT imaging.

Optionally and/or additionally, the mobile positioning device may be configured for supporting a horse having a weight equal or larger than 400 kg, preferably equal or larger than 700 kg, more preferably equal or larger than 1000 kg.

Optionally and/or additionally, the mobile positioning device may comprise a platform extension attachable to the platform, and wherein the platform extension is configured for supporting the body part of the horse to be imaged, preferably wherein the platform extension is receivable through the annular opening of the CT gantry

Optionally and/or additionally, the mobile positioning device may comprise a railing, preferably attachable to the platform.

Optionally and/or additionally, the mobile positioning device may comprise a box-shaped support structure supporting the platform, and wherein the second elevator mechanism is configured for moving the platform in the vertical direction between the lower and upper platform position by moving the box-shaped support structure in the vertical direction, and wherein the movement of the platform in the longitudinal direction with the longitudinal movement mechanism corresponds to a movement of the platform with respect to the box-shaped support structure.

Optionally and/or additionally, the first and second elevator mechanism may be configured such that when the CT gantry is moved to the upper gantry position and the platform is moved to the lower platform position, a separation distance, measured along the vertical direction, between the top surface of the platform and the isocenter of the CT gantry is equal or larger than 110 cm, preferably equal or larger than 130 cm, more preferably equal or larger than 150 cm.

Optionally, the platform may have a cuboid shape, preferably wherein the platform may have a thickness measured in the vertical direction that is equal or larger than 10 cm and equal or smaller than 30 cm.

Optionally and/or additionally, a top surface of the platform may have a rectangular shape.

Optionally and/or additionally, the vertical direction is perpendicular to the longitudinal direction, and/or the lateral direction is perpendicular to the longitudinal direction and the vertical direction.

According to a second aspect of the invention, a method for imaging a body part of a large animal, such as horse, using a CT imaging system as defined above is provided. The method is defined in the appended claims.

### BRIEF DESCRIPTION OF THE FIGURES

The present disclosure will be further explained with reference to the accompanying drawings, in which:

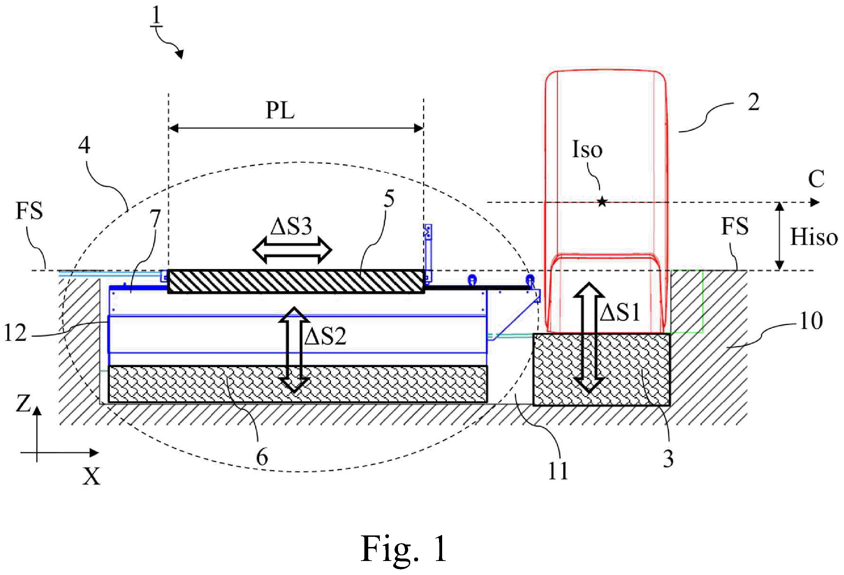

Fig. 1 shows a cross-sectional view of a an embodiment of a CT imaging system according to the present disclosure, wherein the CT gantry has been lowered in the vertical direction such that the CT gantry is located partly below the floor surface FS, and wherein the platform of the mobile positioning device is positioned such that the top surface of the platform is leveled with the floor surface, wherein the cross-sectional view is a cross-section taken in a plane parallel with an X-Z plane defined by axes X and Z, wherein axis X is extending in a longitudinal direction and axis Z is extending in a vertical direction, transverse to the longitudinal direction;

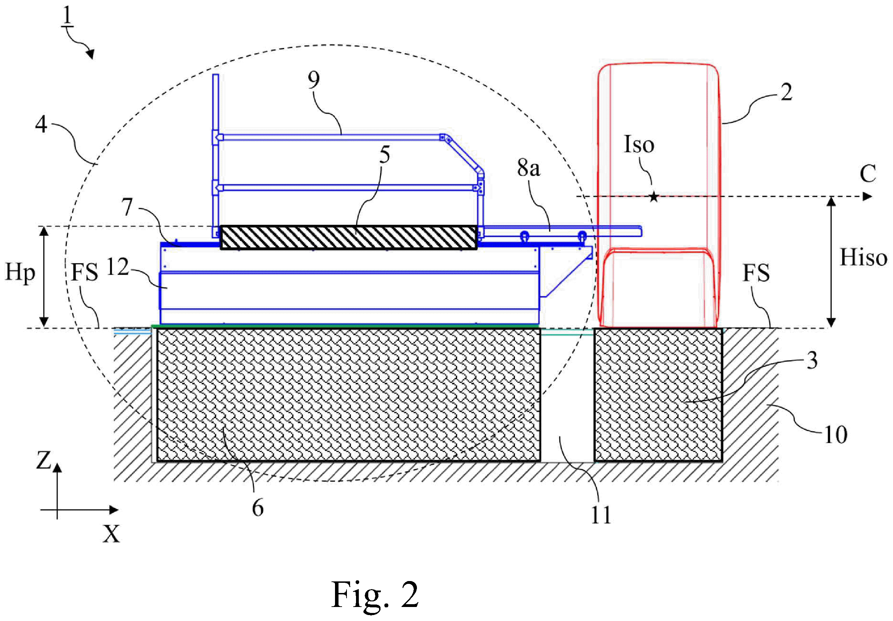

Fig.2 shows a cross-sectional view of the CT imaging system of Fig.1 wherein the height of the CT gantry has been raised in the vertical direction such that the height of the isocenter with respect to the floor level Hiso is increased, and wherein the height of the platform with respect to the floor surface has been raised;

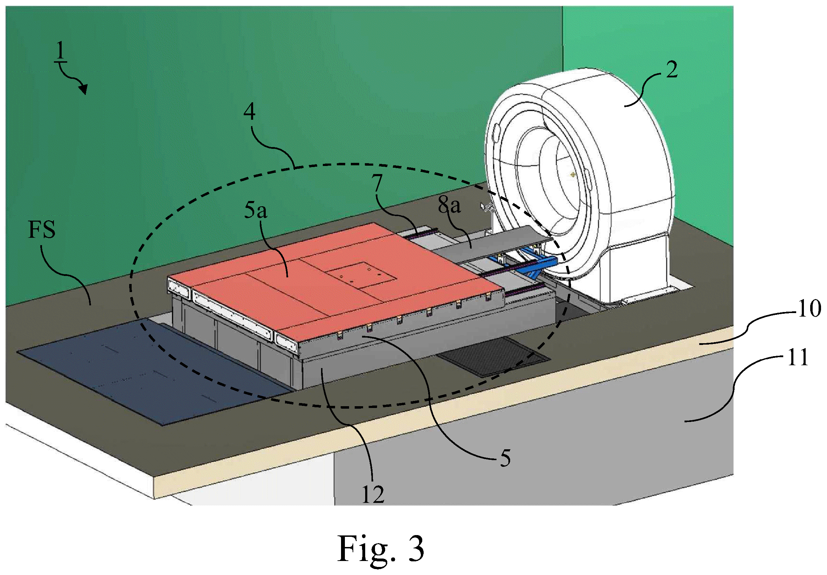

Fig.3 shows an isometric view of an example of a CT imaging system according to present disclosure;

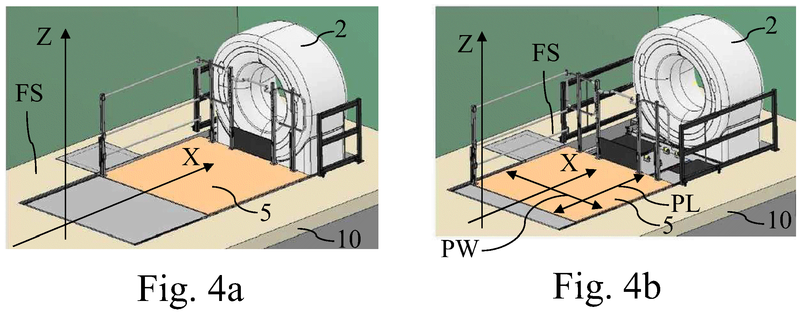

Fig.4a and Fig.4b show an isometric view of an embodiment of a CT imaging system according to the present disclosure wherein the platform of the mobile positioning device is leveled with the floor surface and wherein the CT gantry has been lowered, and wherein Fig.4a and Fig.4b illustrate two different longitudinal positions of the platform;

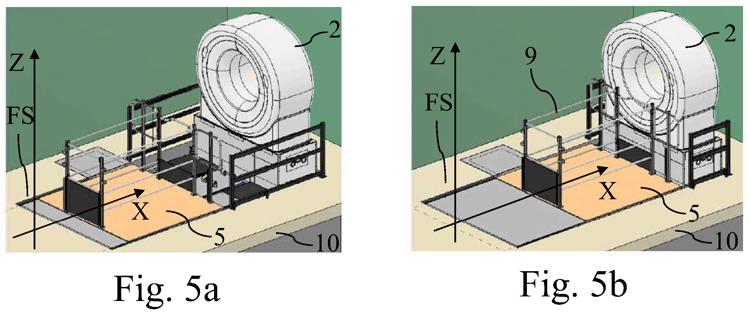

Fig.5a and Fig.5b show an isometric view of the embodiment of a CT imaging system shown on Fig.4a and Fig.4b wherein the height of the platform is maintained leveled with the floor surface and wherein the CT gantry has been raised above the floor level, and wherein Fig.5a and Fig.5b illustrate two different longitudinal positions of the platform;

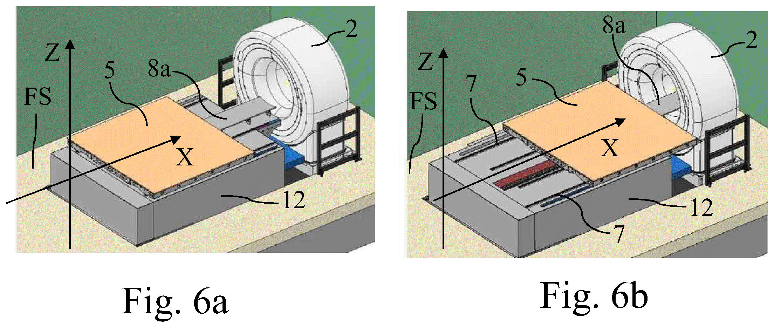

Fig.6a and Fig.6b show an isometric view of the embodiment of a CT imaging system shown on Fig.5a and Fig.5b wherein the height of the platform has been raised above the floor surface for forming a table and wherein the CT gantry has been positioned at an intermediate height position, and wherein Fig.6a and Fig.6b illustrate two different longitudinal positions of the platform;

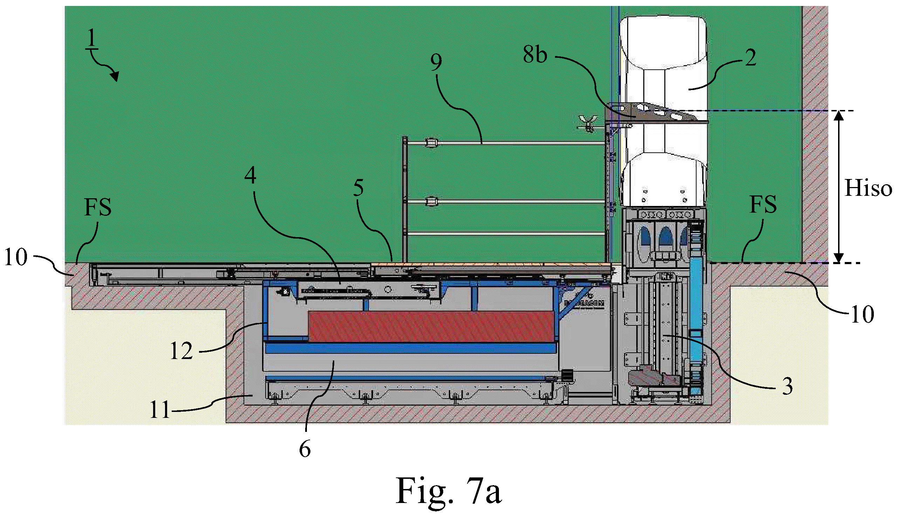

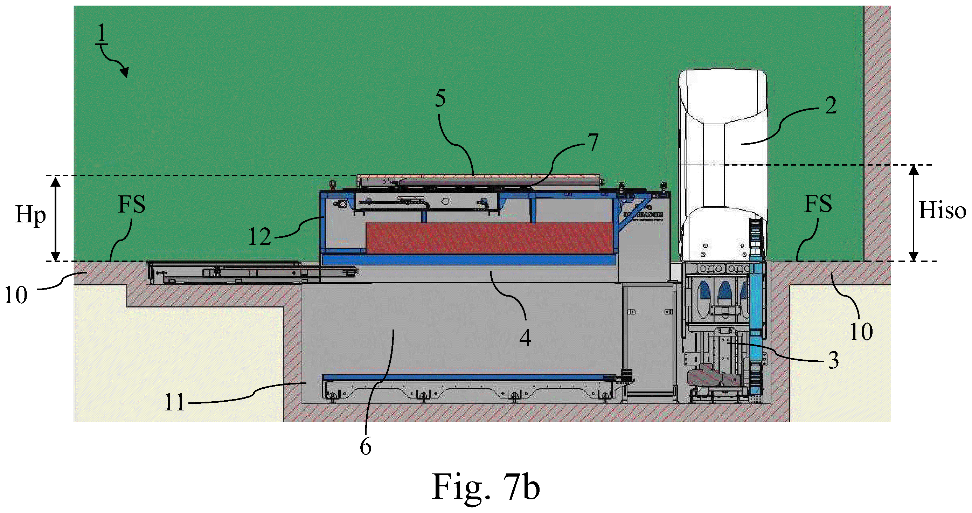

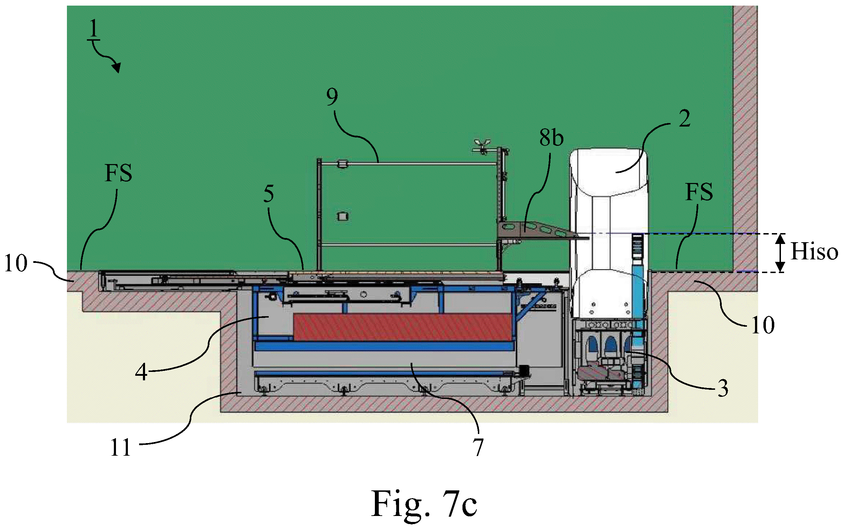

Fig.7a, Fig.7b and Fig.7c show cross-sectional views of an example of a CT imaging system according to the present disclosure for different vertical positions of the CT gantry and the platform with respect to the surface of the floor.

It is noted that the figures are only schematic representations of exemplary embodiments of the invention. Like parts are generally indicated with like reference numerals.

### DETAILED DESCRIPTION

With reference to Fig.1 and Fig.2, cross-sectional views of an embodiment of a computed tomography (CT) imaging system 1 according to the present invention for imaging a body part of a large animal are schematically shown. In Fig.1 and Fig.2, a longitudinal direction is identified with an axis X and a vertical direction is identified with an axis Z. A lateral direction may for example be identified by an axis Y, perpendicular to the axes X and Z.

With reference to Figures 3 to 6b, isometric views are shown of examples of an embodiment of a CT imaging system 1 according to the present disclosure. Figures 7a to 7c show cross-sectional views of an exemplary embodiment of a CT imaging system 1.

More specifically, the CT imaging system 1 according to the present disclosure is a system configured for imaging a body part of a horse. In other words, the CT imaging system according to the present disclosure is at least suitable for performing a CT scan of a body part of a horse. Of course, other large animals such as a pony, a cow, a bear, or any other large animal that might need imaging, may be imaged with the CT imaging system 1 according to the present disclosure, within the load limit accepted by the special table or platform described below.

The CT imaging system comprises a CT gantry 2 having an annular opening for receiving the body part of the animal to be imaged and/or examined.

In embodiments, the CT gantry 2 may be a commercial available CT gantry wherein the annular opening has a large diameter, for instance a diameter between 70 cm and 90 cm.

Typically, as schematically illustrated on Fig.1 and Fig.2, the CT gantry 2 has a central axis C, also named scanning axis, which is extending through the annular opening of the CT gantry 2 and which is crossing the isocenter Iso of the gantry. The isocenter may be considered as a central point of the CT gantry. In Fig.1 and Fig.2, the central axis C is shown to be parallel with the longitudinal direction X.

The CT gantry 2 is to be considered as a classical fixed CT gantry, i.e. the CT gantry 2 is for instance not moveable in the longitudinal direction X, and hence during imaging the CT gantry 2 remains stationary.

Typically, a classical CT gantry comprises at least an X-ray source, e.g. an X-ray tube assembly producing a fan X-ray beam, and a detector for detecting X-rays having crossed the body part under examination. The detector may for instance be a detector array for detecting the fan X-ray beam. This X-ray source and detector are rotatably mounted and the central axis C of the CT gantry generally corresponds to the axis of rotation of the X-ray source and detector pair.

The CT imaging system 1 further comprises a first elevator mechanism 3 that is configured for moving the CT gantry 2 in a vertical direction Z, transverse to a longitudinal direction X, between a lower gantry position and an upper gantry position. In this way, the height of the isocenter Hiso relative to a floor surface FS of a floor 10 is variable. The height of the isocenter Hiso is schematically indicated on Fig.1 and Fig.2 and on Figures 7a to 7c. For example in Fig.1 and Fig.7c, the CT gantry 2 is lowered to a lower gantry position, which is a position wherein the CT gantry 2 is partly lowered below the floor surface FS of the floor 10. On the other hand, in Fig.2 and Fig.7b, the CT gantry 2 is shown to be on the level of the floor surface FS of the floor 10. In Fig.7a, the CT gantry 2 is shown to be raised to its upper position above the floor surface FS of the floor 10.

In embodiments, the vertical direction Z is perpendicular to the longitudinal direction X.

In embodiments, the first elevator mechanism 3 may comprise a plurality of motorized screw jacks. Other elevation mechanism may be considered by the person skilled in the art to move the CT gantry up and down.

The CT imaging system 1 further comprises a mobile positioning device 4 for positioning the large animal during imaging. As schematically shown on Fig.1 to Fig.3, the mobile positioning device 4 is located in front of the CT gantry 2.

As the CT imaging system is for imaging large animals such as horses, the mobile positioning device 4 may be configured for supporting an animal having a weight equal or larger than 400 kg, preferably equal or larger than 700 kg, more preferably equal or larger than 1000 kg.

The mobile positioning device 4 comprising at least a platform 5 configured for supporting the large animal both in a standing position and in a recumbent position. Hence, the size of the platform is dimensioned for supporting both a horse in a standing and a recumbent position. For example, the platform may have a platform length PL measured in the longitudinal direction X that is equal or larger than 1.8 m, preferably equal or larger than 2 m, and the platform may have a platform width PW measured in a lateral direction perpendicular to the longitudinal direction that is equal or larger than 1.2 m, preferably equal or larger than 1.5 m.

In embodiments, the platform 5 may have a cuboid shape.

In embodiments, the platform 5 may for example have a thickness measured in the vertical direction that is equal or larger than 10 cm and equal or smaller than 30 cm.

In embodiments, a top surface of the platform 5 may have a rectangular shape.

The platform 5 of the mobile positioning device 4 can be moved both in the vertical Z as well as in the longitudinal direction X.

Thereto, the mobile positioning device 4 comprises a second elevator mechanism 6 configured for moving the platform 4 in the vertical direction Z between a lower platform position and an upper platform position, and a longitudinal movement mechanism 7 configured for moving the platform 4 in the longitudinal direction X between a first and second longitudinal platform position.

By moving the platform 5 in the vertical direction Z a height of the platform Hp with respect to the floor surface FS is variable. For example, in Fig.2 and Fig.7b, the platform 5 is shown to be raised above the floor level FS. The height Hp of the platform 5 is generally measured with respect to the top surface of platform. In embodiments, the lowest platform position corresponds to a position wherein the top surface of the platform is level with the floor surface FS, as schematically shown on Fig.1, Fig.7a and Fig.7c.

In embodiments, the second elevator mechanism 6 comprises for example a hydraulic lifting device for lifting the platform 5.

The longitudinal movement mechanism 7 may comprise rails for sliding the platform 5 in the longitudinal direction X, as schematically illustrated on Fig.6b.

In embodiments, as shown in more detail on Fig.6a and Fig.6b, the mobile positioning device 4 comprises a box-shaped support structure 12 supporting the platform 5. In these embodiments, the second elevator mechanism 6 is configured for moving the platform 5 in the vertical direction Z between the lower and upper platform position by moving the box-shaped support structure 12 in the vertical direction. Further, the movement of the platform 5 in the longitudinal direction X with the longitudinal movement mechanism 7 corresponds to a movement of the platform with respect to the box-shaped support structure.

In other words, the first and second elevator mechanism are configured for operating independently from each other such that the height of the isocenter of the CT gantry and the height of the platform are independently adjustable. In this way, by adequately defining a height position of the platform and an isocenter position of the CT gantry, a large number of body parts of the horse may be receivable at the isocenter of the gantry and hence may be imaged.

To be able to image both the head and feet of a horse, the height of the isocenter Hiso of the CT gantry relative to the floor surface FS is generally variable by at least 80 cm, preferably by at least 100 cm.

The travel stroke of the first 3 and second 6 elevator mechanism may be selected to be able to image both head and feet of a horse or poney.

In embodiments, the first 3 elevator mechanism may have a first travel stroke ΔS1 in the vertical direction for moving the CT gantry between the lower and upper gantry position. The first travel stroke ΔS1 is for example equal or larger than 30 cm, preferably equal or larger than 50 cm, more preferably equal or larger than 80 cm.

In embodiments, the first travel stroke ΔS1 for moving the CT gantry may be equal or larger than 100 cm or even larger, for example 120 cm.

The second elevator mechanism 6 may have a second travel stroke ΔS2 in the vertical direction for moving the platform 5 between the lower and upper platform position. The second travel stroke ΔS2 is for example equal or larger than 70 cm, preferably equal or larger than 80 cm, more preferably equal or larger than 90 cm.

In embodiments, depending on the travel stroke of the second elevator mechanism, the lower platform position may corresponds to a position wherein a top surface of the platform is leveled with the floor surface FS.

In alternative embodiments, the lower platform position may corresponds to a position wherein the top surface of the platform is lower than the floor surface.

In embodiments, the platform 5 may comprise a cushion 5a, and wherein the top surface of the platform is formed by a surface of the cushion 5a.

The floor surface FS, schematically indicated on Fig.1 to Fig.7c, has to be interpreted as a surface associated to a floor of a room wherein the CT imaging system 1 is located. Generally the floor surface FS is a walking surface that allows persons to walk around the mobile positioning device 4 and/or the CT gantry.

In embodiments, the longitudinal movement mechanism 7 has a third travel stroke ΔS3 for moving the platform in the longitudinal direction X between a first and a second platform longitudinal position. This third travel stroke ΔS3 is for example equal or larger than 50 cm, preferably equal or larger than 70 cm, more preferably equal or larger than 90 cm. In some embodiments the third travel stroke ΔS3 may even be equal or larger than 130 cm.

As illustrated in Fig.7a to Fig.7c, a pit 11 may be provided in the floor and the first and/or second elevator mechanism may be located in the pit 11 in the floor of the room where the CT imaging system 1 is installed.

The CT imaging system further comprises a control system for controlling the movement mechanisms and for controlling image acquisition.

In embodiments, in one operational mode, an operator may, through a user interface, manually control the vertical movement of the CT gantry and/or the vertical and longitudinal movement of the mobile positioning device.

In embodiments, for instance when in an imaging mode, the control system may be configured for taking a CT scan, e.g. a helical CT scan, by synchronizing a movement of the platform 5 along the longitudinal direction X with a rotational movement of the X-ray source and/or detector. This movement of the platform in the longitudinal direction X during imaging is established with the longitudinal movement mechanism 7, as discussed above. From a control point of view, this is a similar approach as with a CT scanner for humans wherein a movement of a patient table is to be synchronized with the rotation of the X-ray source and/or detector for taking a CT scan. Hence, to establish such a synchronization, control systems and electronics known in the art from fixed gantry CT scanners may be applied or be adapted for use with the present mobile positioning device 4 having a platform 5, and wherein the platform 5 supporting the horse, is to be moved in the longitudinal direction during performance of a CT scan.

As a commercial CT gantry may be used for the CT imaging system according to the present disclosure, the CT gantry 2 is equipped with a standard X-ray system known in the art, and typically comprise for example an X-ray tube as an X-ray source and a solid-state scintillation detector array as a detector for detecting the X-rays. The X-ray source and detector are rotatably mounted in the gantry.

In some embodiments, the mobile positioning device may comprise a platform extension 8a, 8b attachable to the platform 5. Such platform extension is configured for supporting the body part of the large animal to be imaged. In embodiments, the platform extension is receivable through the annular opening of the CT gantry.

For example, if a head of the horse needs imaging, the horse will be standing on the platform 5 and put his head through the opening of the CT scanner 2. In this example, the platform extension may be a head extension 8b, schematically shown on Fig.7a, allowing the horse to rest his head on the head extension during imaging. The head extension 8b may be rigidly fixed to the platform such that when the platform is moving in the longitudinal direction, also the head extension is moving in the longitudinal direction together with the platform.

In a further example, if for example the horse is in recumbent position, under anesthesia, and a limb part needs imaging, the platform extension may be in the form of a table extension 8a, as schematically shown on Fig.2 and Fig.3, that allows to support the limb on the table extension. Also in this example the table extension 8a is rigidly fixed to the platform such that when the platform is moved in the longitudinal direction, also the table extension 8a is moved in the longitudinal direction. This table extension 8a is dimensioned such that it is receivable through the opening of the CT gantry.

In some embodiments, as schematically shown on Fig.2, Fig.7a and Fig.7c, the mobile positioning device may further comprises a railing 9 attachable to the platform 5 to facilitate the positioning of the horse on the platform.

With reference to Fig.4a to Fig.6b isometric views of an embodiment of a CT imaging system 1 according to the present disclosure are shown wherein the platform 5 of the mobile positioning device 4 and/or CT gantry 2 are positioned in different positions.

For example in Fig.4a and Fig.4b the platform 5 of the mobile positioning device 4 is leveled with the floor surface FS and the CT gantry 2 has been lowered when compared to its upper position. In Fig.4a and Fig.4b two different longitudinal positions of the platform 5 are illustrated, in Fig.4a, the platform 5 is moved towards the CT gantry 2, while in Fig.4b, the platform 5 is retracted in the longitudinal direction X from the CT gantry 2.

In Fig.5a and Fig.5b, the height of the platform 5 is maintained leveled with the floor surface FS of the floor 10 but the CT gantry 2 has been raised above the floor level towards the upper gantry position. In Fig.5a the platform 5 is retracted from the CT gantry, while in Fig.5b the platform 5 is moved in the longitudinal direction X towards the CT gantry 2.

In Fig.6a and Fig.6b the height of the platform 5 has been raised above the floor surface for forming a table, e.g. for resting a horse under anesthesia, and wherein the CT gantry 2 has been positioned at an intermediate height position. Advantageously, in this example, a platform extension 8a is coupled to the platform 5. Also, in this example, Fig.6a and Fig.6b illustrate two different longitudinal positions for the platform 5.

The CT imaging system 1 according to the present disclosure also allows that, when the platform 5 is at the floor level, an animal lying on a surgical table can be brought there for performing complementary examinations, in particular outside the area supported by the table.

In embodiments, the longitudinal, lateral and vertical direction are defined by axes X,Y,Z, which may for example form an orthogonal coordinate system.

According to a further aspect of the present disclosure, a method for imaging a body part of a large animal, such as horse, is provided using a CT imaging system 1 as discussed above. The method comprises steps of:
- positioning the animal on the platform 5 in a standing position or in a recumbent position,
- adjusting and/or setting the height of the isocenter Hiso relative to a floor surface FS by using the first elevator mechanism 3 configured for moving the CT gantry 2 in the vertical direction Z, transverse to the longitudinal direction X, preferably wherein the height of the isocenter is adjusted and/or set to a first predefined height,
- adjusting and/or setting the height of the platform Hp relative to the floor surface by using the second elevator mechanism 6 configured for moving the platform 5 in the vertical direction, preferably wherein the height of the platform 5 is adjusted and/or set to a second predefined height, and
- performing CT imaging.

In embodiments, the method may further comprise a step of:
- using a platform extension 8a, 8b attached to the platform 5 for supporting the body part of the large animal to be imaged, and adjusting the height and/or the longitudinal position of the platform 5 such that the platform extension 8a, 8b is received through the annular opening of the CT gantry 2.

In embodiments, as discussed above, a CT scan, e.g. a helical CT scan, may be made. Thereto, the method may further comprises a step of: -performing CT imaging by moving the platform along the longitudinal direction X synchronized with a rotational movement of the X-ray source and/or the detector for detecting X-rays.

As mentioned above, in some cases, when the platform is at the floor level, positioning the animal might involve: bringing the animal while lying onto a surgical table. This allows to perform complementary examinations while the animal remains lying on the surgical table.

The present disclosure has been described in terms of specific embodiments, which are illustrative of the disclosure and not to be construed as limiting. It will be appreciated by persons skilled in the art that the present disclosure is not limited by what has been particularly shown and/or described and that alternatives or modified embodiments could be developed in the light of the overall teaching of this disclosure. The drawings described are only schematic and are non-limiting.

Use of the verb "to comprise", as well as the respective conjugations, does not exclude the presence of elements other than those stated. Use of the article "a", "an" or "the" preceding an element does not exclude the presence of a plurality of such elements.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the disclosure described herein are capable of operation in other sequences than described or illustrated herein.

Reference throughout this specification to "one embodiment" or "an embodiment" means that a particular feature, structure or characteristic described in connection with the embodiments is included in one or more embodiment of the present disclosure. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one ordinary skill in the art from this disclosure, in one or more embodiments.

### CLAUSES

Here below, text is provided in the form of clauses. The clauses comprise characterizations indicating a variety of options, features, and feature combinations that can be used in accord with the teachings of the present disclosure. Alternate characterizations of the ones given, but consistent with the descriptions here above, are possible. In summary, according to the present disclosure, the following clauses could for instance be claimed:
1. A CT imaging system (1) for imaging a body part of a large animal, comprising:
   - a CT gantry (2) having an annular opening for receiving the body part to be imaged and/or examined, and wherein a central axis (C) of the CT gantry extending through said opening is crossing an isocenter (Iso) of the CT gantry, and wherein the central axis is parallel with a longitudinal direction (X),
   - a first elevator mechanism (3) configured for moving the CT gantry in a vertical direction (Z), transverse to the longitudinal direction (X), between a lower CT gantry position and an upper CT gantry position, such that a height of the isocenter (Hiso) relative to a floor surface (FS) is variable,
   - a mobile positioning device (4) for positioning the large animal, comprising at least:
      a) a platform (5) configured for supporting the large animal in a standing position and in a recumbent position,
      b) a second elevator mechanism (6) configured for moving the platform (5) in the vertical direction (Z) between a lower platform position and an upper platform position, such that a height of the platform (Hp) with respect to the floor surface (FS) is variable, and
      c) a longitudinal movement mechanism (7) configured for moving the platform (5) in the longitudinal direction (X).
2. The CT imaging system according to clause 1 wherein the first elevator mechanism has a first travel stroke (ΔS1) in the vertical direction for moving the CT gantry between the lower and upper gantry position, and wherein said first travel stroke (ΔS1) is equal or larger than 30 cm, preferably equal or larger than 50 cm, more preferably equal or larger than 80 cm.
3. The CT imaging system according to clause 1 or clause 2 wherein the second elevator mechanism has a second travel stroke (ΔS2) in the vertical direction for moving the platform between the lower and upper platform position, and wherein said second travel stroke (ΔS2) is equal or larger than 70 cm, preferably equal or larger than 80 cm, more preferably equal or larger than 90 cm.
4. The CT imaging system according to any of previous clauses wherein the longitudinal movement mechanism (7) has a third travel stroke (ΔS3) for moving the platform in the longitudinal direction between a first and a second platform longitudinal position, and wherein said third travel stroke (ΔS3) is equal or larger than 50 cm, preferably equal or larger than 70 cm, more preferably equal or larger than 130 cm.
5. The CT imaging system according to any of previous clauses wherein said lower platform position corresponds to a position wherein a top surface of the platform is leveled with the floor surface, or alternatively wherein the top surface of the platform is lower than the floor surface.
6. The CT imaging system according to any of previous clauses wherein said upper platform position corresponds to a position wherein the top surface of the platform is raised by at least 70 cm, preferably by at least 80 cm, more preferably by at least 90 cm above the floor surface.
7. The CT imaging system according to any of previous clauses wherein the first and second elevator mechanism are configured for operating independently from each other such that said height of the isocenter of the CT gantry and said height of the platform are independently adjustable.
8. The CT imaging system according to any of previous clauses wherein the platform has a platform length (PL) measured in the longitudinal direction (X) that is equal or larger than 1.8 m, preferably equal or larger than 2 m, and wherein the platform has a platform width (PW) measured in a lateral direction perpendicular to the longitudinal direction (X) that is equal or larger than 1.2 m, preferably equal or larger than 1.5 m.
9. The CT imaging system according to any of previous clauses wherein the platform has a cuboid shape, preferably wherein the platform has a thickness measured in the vertical direction that is equal or larger than 10 cm and equal or smaller than 30 cm.
10. The CT imaging system according to any of previous clauses wherein the first elevator mechanism (3) comprises a plurality of screw jacks, preferably wherein the screw jacks are motorized.
11. The CT imaging system according to any of previous clauses wherein the second elevator mechanism (6) comprises a hydraulic lifting device.
12. The CT imaging system according to any of previous clauses wherein the mobile positioning device (4) comprises a box-shaped support structure (12) supporting the platform (5), and wherein the second elevator mechanism is configured for moving the platform (5) in the vertical direction (Z) between the lower and upper platform position by moving the box-shaped support structure (12) in the vertical direction, and wherein said movement of the platform (5) in the longitudinal direction (X) with the longitudinal movement mechanism (7) corresponds to a movement of the platform with respect to said box-shaped support structure.
13. The CT imaging system according to any of previous clauses wherein the first (3) and second (6) elevator mechanism are configured such that when the CT gantry is moved to the upper gantry position and the platform is moved to the lower platform position, a separation distance, measured along the vertical direction, between the top surface of the platform and the isocenter of the CT gantry is equal or larger than 110 cm, preferably equal or larger than 130 cm, more preferably equal or larger than 150 cm.
14. The CT imaging system according to any of previous clauses wherein the CT gantry (2) is not moveable in the longitudinal direction (X).
15. The CT imaging system according to any of previous clauses wherein the CT gantry (2) comprises at least an X-ray source and a detector for detecting X-rays, and wherein said X-ray source and detector are rotatably mounted, preferably wherein said central axis is an axis of rotation for said X-ray source and detector.
16. The CT imaging system according to clause 15 further comprising a control system configured for taking a CT scan, such as for example a helical CT scan, by synchronizing a movement of the platform (5) along the longitudinal direction (X) with a rotational movement of the X-ray source and detector.
17. The CT imaging system according to any of previous clauses wherein the mobile positioning device is configured for supporting an animal having a weight equal or larger than 400 kg, preferably equal or larger than 700 kg, more preferably equal or larger than 1000 kg.
18. The CT imaging system according to any of previous clauses wherein the CT imaging system is a system for imaging a body part of a horse.
19. The CT imaging system according to any of previous clauses wherein the floor surface is a surface associated to a floor of a room wherein the computed tomography imaging system is located.
20. The CT imaging system according to clause 19 wherein the first and second elevator mechanism are located in a pit (11) in the floor of the room.
21. The CT imaging system according to any of previous clauses wherein the mobile positioning device (4) is located in front of the CT gantry (2).
22. The CT imaging system according to any of previous clauses wherein the height of the platform with respect to the floor surface is measured with respect to the top surface of the platform.
23. The CT imaging system according to any of previous clauses wherein the mobile positioning device comprises a platform extension (8a, 8b) attachable to the platform (5), and wherein the platform extension is configured for supporting the body part of the large animal to be imaged.
24. The CT imaging system according to clause 23 wherein the platform extension is receivable through the annular opening of the CT gantry.
25. The CT imaging system according to any of previous clauses wherein the mobile positioning device (4) comprises a railing (9), preferably attachable to the platform.
26. The CT imaging system according to any of previous clauses wherein a top surface of the platform (5) has a rectangular shape.
27. A method for imaging a body part of a large animal, such as horse, using a CT imaging system, wherein the CT imaging system comprises at least a CT gantry (2) and a platform (5) in front of the CT gantry for supporting a large animal in a standing position and in a recumbent position, and wherein the CT gantry has an annular opening for receiving the body part to be imaged, and wherein a central axis of the CT gantry extending through the opening is crossing an isocenter of the CT gantry, and wherein the central axis is parallel with a longitudinal direction (X), the method comprising:
   - positioning the animal on the platform in a standing position or in a recumbent position,
   - adjusting a height of the isocenter (Hiso) relative to a floor surface (FS) by using a first elevator mechanism (3) configured for moving the CT gantry in a vertical direction (Z), transverse to the longitudinal direction (X), preferably wherein the height of the isocenter is a first predefined height,
   - adjusting a height of the platform (Hp) relative to the floor surface by using a second elevator mechanism (6) configured for moving the platform (4) in the vertical direction, preferably wherein the height of the platform is a second predefined height, and
   - performing CT imaging.
28. The method of clause 27 further comprising:
   - using a platform extension (8a, 8b) attached to the platform for supporting the body part of the large animal to be imaged, and adjusting the height and /or longitudinal position of the platform such that the platform extension is received through the annular opening of the CT gantry (2).
29. The method of any of clauses 27 to 28 wherein the position of CT gantry remains stationary during performance of the CT imaging.
30. The method of any of clauses 27 to 29 comprising:
   -performing CT imaging by moving the platform along the longitudinal direction synchronized with a rotational movement of an X-ray source and detector of the CT gantry, so as to obtain a helical CT scan.

### REFERENCE NUMBERS

| | |
|---|---|
| 1 | CT imaging system |
| 2 | CT gantry |
| 3 | First elevator mechanism |
| 4 | Mobile positioning device |
| 5 | platform |
| 5a | cushion |
| 6 | Second elevator mechanism |
| 7 | Longitudinal movement mechanism |
| 8a, 8b | Platform extension |
| 9 | Platform railing |
| 10 | floor |
| 11 | pit |
| 12 | Box-shaped support structure |
| C | Central axis CT gantry |
| FS | Floor surface |
| Hiso | Height isocenter |
| iso | isocenter |
| PL | Platform length |
| PW | Platform width |
| X | Longitudinal direction |
| Z | Vertical direction |
| Y | Lateral direction |

## Claims

1. A CT imaging system (1) for imaging a body part of a horse, comprising:
- a CT gantry (2) having an annular opening for receiving the body part to be imaged, wherein a central axis (C) of the CT gantry extending through said opening is crossing an isocenter (Iso) of the CT gantry, and wherein the central axis is parallel with a longitudinal direction (X), and wherein the CT gantry (2) comprises at least an X-ray source and a detector for detecting X-rays,
- a first elevator mechanism (3) configured for moving the CT gantry in a vertical direction (Z), transverse to the longitudinal direction (X), between a lower CT gantry position and an upper CT gantry position, such that a height of the isocenter (Hiso) relative to a floor surface (FS) is variable,
- a mobile positioning device (4) for positioning the horse, comprising at least:
a) a platform (5) configured for supporting the horse in a standing position and in a recumbent position, wherein the platform has a platform length (PL) measured in the longitudinal direction (X) that is equal or larger than 1.8 m, preferably equal or larger than 2 m, and wherein the platform has a platform width (PW) measured in a lateral direction perpendicular to the longitudinal direction (X) that is equal or larger than 1.2 m, preferably equal or larger than 1.5 m,
b) a second elevator mechanism (6) configured for moving the platform (5) in the vertical direction (Z) between a lower platform position and an upper platform position, such that a height of the platform (Hp) with respect to the floor surface (FS) is variable, and
c) a longitudinal movement mechanism (7) configured for moving the platform (5) in the longitudinal direction (X), and
- a control system configured for taking a CT scan by synchronizing a movement of the platform (5) along the longitudinal direction (X) with a rotational movement of the X-ray source and/or the detector for detecting X-rays.

2. The CT imaging system according to claim 1,
wherein the first elevator mechanism (3) has a first travel stroke (ΔS1) in the vertical direction for moving the CT gantry between the lower and upper gantry position, and wherein said first travel stroke (ΔS1) is equal or larger than 30 cm, preferably equal or larger than 50 cm, more preferably equal or larger than 80 cm,
wherein the second elevator mechanism (6) has a second travel stroke (ΔS2) in the vertical direction for moving the platform between the lower and upper platform position, and wherein said second travel stroke (ΔS2) is equal or larger than 70 cm, preferably equal or larger than 80 cm, more preferably equal or larger than 90 cm, and
wherein the longitudinal movement mechanism (7) has a third travel stroke (ΔS3) for moving the platform in the longitudinal direction between a first and a second platform longitudinal position, and wherein said third travel stroke (ΔS3) is equal or larger than 50 cm, preferably equal or larger than 70 cm, more preferably equal or larger than 130 cm.

3. The CT imaging system according to any of previous claims wherein said lower platform position corresponds to a position wherein a top surface of the platform (5) is leveled with the floor surface or wherein the top surface of the platform (5) is lower than the floor surface.

4. The CT imaging system according to any of previous claims wherein said upper platform position corresponds to a position wherein the top surface of the platform is raised by at least 70 cm, preferably by at least 80 cm, more preferably by at least 90 cm above the floor surface.

5. The CT imaging system according to any of previous claims wherein the first and second elevator mechanism are configured for operating independently from each other such that said height of the isocenter of the CT gantry and said height of the platform are independently adjustable.

6. The CT imaging system according to any of previous claims wherein the platform has a cuboid shape, preferably wherein the platform has a thickness measured in the vertical direction that is equal or larger than 10 cm and equal or smaller than 30 cm.

7. The CT imaging system according to any of previous claims wherein the first elevator mechanism (3) comprises a plurality of screw jacks, preferably wherein the screw jacks are motorized and/or wherein the second elevator mechanism (6) comprises a hydraulic lifting device.

8. The CT imaging system according to any of previous claims wherein the mobile positioning device (4) comprises a box-shaped support structure (12) supporting the platform (5), and wherein the second elevator mechanism is configured for moving the platform (5) in the vertical direction (Z) between the lower and upper platform position by moving the box-shaped support structure (12) in the vertical direction, and wherein said movement of the platform (5) in the longitudinal direction (X) with the longitudinal movement mechanism (7) corresponds to a movement of the platform with respect to said box-shaped support structure.

9. The CT imaging system according to any of previous claims wherein the first (3) and second (6) elevator mechanism are configured such that when the CT gantry (2) is moved to the upper gantry position and the platform is moved to the lower platform position, a separation distance, measured along the vertical direction, between the top surface of the platform and the isocenter of the CT gantry is equal or larger than 110 cm, preferably equal or larger than 130 cm, more preferably equal or larger than 150 cm.

10. The CT imaging system according to any of previous claims wherein the CT gantry (2) is not moveable in the longitudinal direction (X), preferably wherein during said taking of a CT scan, the CT gantry remains stationary.

11. The CT imaging system according to any of previous claims, wherein said central axis of the CT gantry (2) is an axis of rotation for said X-ray source and/or detector.

12. The CT imaging system according to any of previous claims wherein the floor surface (FS) is a surface associated to a floor of a room wherein the computed tomography imaging system is located, and wherein the first and second elevator mechanism are located in a pit (11) in the floor of the room.

13. The CT imaging system according to any of previous claims wherein the mobile positioning device comprises a platform extension (8a, 8b) attachable to the platform (5), and wherein the platform extension is configured for supporting the body part of the horse to be imaged, preferably wherein the platform extension is receivable through the annular opening of the CT gantry.

14. A method for imaging a body part of a large animal, such as horse, using a CT imaging system (1) according to any of previous claims, the method comprising:
- positioning the animal on the platform (5) in a standing position or in a recumbent position,
- adjusting and/or setting the height of the isocenter (Hiso) relative to the floor surface (FS) by using the first elevator mechanism (3) for moving the CT gantry in the vertical direction (Z), preferably wherein the height of the isocenter is adjusted and/or set to a first predefined height,
- adjusting and/or setting the height of the platform (Hp) relative to the floor surface by using the second elevator mechanism (6) for moving the platform (5) in the vertical direction, preferably wherein the height of the platform is adjusted and/or set to a second predefined height, and
- performing CT imaging by moving the platform (5) along the longitudinal direction synchronized with a rotational movement of the X-ray source and/or the detector for taking X-rays, preferably wherein the position of CT gantry remains stationary during performance of the CT imaging.

15. The method of claim 14 further comprising:
- using a platform extension (8a, 8b) attached to the platform (5) for supporting the body part of the large animal to be imaged, and adjusting the height and/or the longitudinal position of the platform (5) such that the platform extension (8a, 8b) is received through the annular opening of the CT gantry(2).
